# EUROPEAN PATENT SPECIFICATION

(11) **EP 1 351 979 B1**
(45) Date of publication and mention of the grant of the patent: **16.01.2008**
(21) Application number: 01988426.1
(22) Date of filing: 19.12.2001
(51) Int. Cl.: C07K 14/00, C12P 19/34

(54) **REGULATED ACTIVATION OF CELL-MEMBRANE RECEPTORS BY METAL-CHELATING AGONISTS**
REGULIERTE AKTIVIERUNG VON ZELLMEMBRANREZEPTOREN DURCH METALLCHELATISIERENDE AGONISTEN
ACTIVATION REGULEE DES RECEPTEURS DE LA MEMBRANE CELLULAIRE AU MOYEN D'AGONISTES CHELATEURS DES METAUX

(30) Priority: 21.12.2000 US 257002 P
(43) Date of publication of application: 15.10.2003
(73) Proprietor: SMITHKLINE BEECHAM CORPORATION, Philadelphia, PA 19406 (US); Ligand Pharmaceuticals, San Diego, CA 92121 (US)
(72) Inventor: DELORME, Evelyn, O., Ligand Pharmaceuticals, San Diego, CA 92121 (US); DUFFY, Kevin, J., GlaxoSmithKline, Collegeville, PA 19426 (US); LAMB, Peter, I., Ligand Pharmaceuticals, San Diego, CA 92121 (US); LUENGO, Juan, I., GlaxoSmithKline, Collegeville, PA 19426 (US); TIAN, Shin-Shay, C., Ligand Pharmaceuticals, San Diego, CA 92121 (US)
(74) Representative: Breen, Anthony Paul
(86) International application number: PCT/US2001/050777
(87) International publication number: WO 2002/057300

(56) References cited:
- US-A- 6 140 120
- NORREGAARD L ET AL: "Structural probing of a microdomain in the dopamine transporter by engineering of artificial Zn2+ binding sites." BIOCHEMISTRY. 26 DEC 2000, vol. 39, no. 51, 30 November 2000 (2000-11-30), pages 15836-15846, XP002319728 ISSN: 0006-2960
- UENO S ET AL: "Tryptophan scanning mutagenesis in TM2 of the GABA(A) receptor alpha subunit: effects on channel gating and regulation by ethanol." BRITISH JOURNAL OF PHARMACOLOGY. SEP 2000, vol. 131, no. 2, September 2000 (2000-09), pages 296-302, XP001205469 ISSN: 0007-1188
- KOLTCHINE V.: 'Agonist gating and isoflurane potentiation in the human gamma-aminobutyric acid type A receptor determined by the volume of a second transmembrane domain residue' MOLECULAR PHARMACOLOGY vol. 56, 1999, pages 1087 - 1093, XP002950112
- SCHREIBER S.: 'Chemical genetics resulting from a passion for synthetic organic chemistry' BIOORGANIC AND MEDICINAL CHEMISTRY vol. 6, 1998, pages 1127 - 1152, XP002950111
- WEIDERRECHT G.: 'Characterization of high molecular weight FK-506 binding activities reveals a novel FK-506-binding protein as well as a protein complex' J. BIOL. CHEM. vol. 267, no. 30, 25 October 1992, pages 21753 - 21760, XP002950110

## Description

### FIELD OF THE INVENTION

This invention relates to a method to regulate the activation of cell-surface receptors with small molecule agonists by engineering specific point mutations in the transmembrane domains of the receptors. More specifically, the invention describes a method to promote the oligomerization of mutated multimeric receptors using compounds that can chelate metal ions such as zinc (II). Transfection of these modified receptors into host cells provides numerous therapeutic opportunities in gene therapy and other applications related to inducible signal transduction in transduced cells.

### BACKGROUND OF THE INVENTION

Dimerization and oligomerization of cell-surface receptors is a key biological process by which extracellular molecules can regulate diverse biological responses within the cell such as proliferation, differentiation or apoptosis. This signaling mechanism is utilized by many soluble proteins, such as cytokines, hormones and growth factors, which exert their biological functions through the interaction and subsequent aggregation of specific cell-surface receptors. (Arai, K.-I. et al. Annu. Rev. Biochem. 1990, 59, 783; Bazan, J. F. Proc. Natl. Acad. Sci. U.S.A. 1990, 87, 6934; Ullrich A. and Schlessinger, J. Cell, 1990, 61, 203-212; Young, P. R. "Protein hormones and their receptors", Curr. Opin. Biotech. 1992, 3, 408-421; Heldin, C. H., "Dimerization of cell surface receptors in signal transduction" Cell, 1995, 80, 213-223). These receptors are comprised of three distinct domains: an extracellular ligand-binding domain, a transmembrane domain and a cytoplasmic domain, which is responsible for signal transduction within the cell. Receptor dimerization is the first step in a signaling cascade that is mediated by receptor-associated tyrosine kinases. These kinases are activated by autophosphorylation and, in turn, phosphorylate a number of other intracellular targets, such as the cytoplasmic domain of the receptor, adapter proteins and STATs (signal transducers and activators of transcription). The tyrosine-phosphorylated proteins propagate the signaling cascade by acting as binding sites for other intracellular proteins, a process that ultimately results in the initiation of transcription of the specific responsive genes. Some receptors, such as those for thrombopoietin (TPO), granulocyte-colony stimulating factor (G-CSF) erythropoietin (EPO) and growth hormone consist of a single polypeptide subunit. Others, such as receptors for interleukin-2 (IL-2), IL-3, IL-4, IL-5 and IL-6, consist of two or three different chains each performing more specialized functions, such as ligand binding and signal transduction. Although the mechanism of receptor activation varies for specific receptor-ligand pairs, a common feature of many single-transmembrane receptors appears to be their aggregation on the cell membrane in response to binding of their specific ligands. This aggregation event can be in the form of homodimerization, in the case of receptors with a single subunit, or heterodimerization, in the case of receptors with different subunits.

Recently, technology has been developed that allows the dimerization of chimeric cell-membrane receptors by dimeric forms of small molecule ligands derived from FK-506 or cyclosporin A (reviewed in Schreiber, Biorg. Med Chem. 1998, 6, 1127). In this work FK-1012, a lipid-soluble dimeric form of FK-506, is used to dimerize chimeric protein consisting of a cell-membrane receptor signaling domain fused to an FKBP12 domain. The ability of FK-1012 to homodimerize this fusion protein is based on the strong affinity between FK-506, a natural macrocyclic product, and FKBP12, an intracellular cytoplasmic protein present in all cells (Bierer et al. PNAS, 1990, 87, 9231). This methodology has been applied to the intracellular domains of a number of trans-membrane receptors, such as the zeta chain of the T-cell receptor (Spencer et al., Science 1993, 262, 1019; Pruschy et al. Chem. Biol. 1994, 1, 164), Fas receptor (Belshaw et al. Chem. Biol. 1996, 3, 731; Spencer et al., Current Biol 1996, 6, 839), TGF-beta receptor (Spencer et al., Current Biol 1998, 8, 761), EPO receptor (Blau et al. PNAS, 1997 94, 3076), c-kit receptor (Jin et al. Blood 1998, 91, 890). Full reports describing the applications of this technology to the regulation of transcription have recently appeared (US 6140120, US 6063625, US 6054436, US 6046047, US 6043082, US 6011018, US 5994313, US 5871753, US 5869337, US 5834266, US 5830462).

All of the reports listed above involve the construction of chimeric transmembrane proteins consisting of the fusion between a cytoplasmic domain, which contains the signal transduction signal, and a ligand-binding domain, either derived from FKBP12 or cyclophilin. None of the reports suggest a method whereby a transmembrane receptor can be made responsive to the action of small-molecule activators through specific point mutations in their transmembrane domain.

As disclosed herein it has unexpectedly been discovered that by simple mutation of two specific residues within their transmembrane domain, cell-membrane receptors can be specifically activated by small-molecule metal-chelated ligands, such as those described in PCT/US98/23049, published as International Application No. WO 99/22732 on May 14, 1999.

### SUMMARY OF THE INVENTION

Accordingly, one aspect of the present invention is a method for activating dimeric or oligomeric cell-surface receptors which comprises mutating a specific amino acid within the transmembrane domain of the receptor to histidine and thereafter contacting the mutated cell-surface receptor with a metal-chelating receptor agonist.

Another aspect of the present invention is a method for activating dimeric or oligomeric cell-surface receptors which comprises mutating two specific amino acids, preferably three residues apart, within the transmembrane domain of the receptor, to threonine and histidine and thereafter contacting the mutated dimeric cell-surface receptor with a metal-chelating receptor agonist.

Another aspect of the invention relates to dimeric or oligomeric cell-surface receptors containing one point mutation of a specific amino acid within the transmembrane domain of the receptor to histidine.

Another aspect of the invention relates dimeric or oligomeric cell-surface receptors containing two point mutations of specific amino acids, preferably three residues appart, within the transmembrane domain of the receptor to threonine and histidine.

Another aspect of the invention relates to a host cell having dimeric or oligomeric cell-surface receptors containing one point mutation of a specific amino acid within the transmembrane domain of the receptor to histidine.

Another aspect of the invention relates to a host cell having dimeric or oligomeric cell-surface receptors containing two point mutations of a specific amino acids, preferably three residues appart, within the transmembrane domain of the receptor to threonine and histidine.

### DETAILED DESCRIPTION OF THE INVENTION

By the term "heteroatom(s)", as used herein is meant nitrogen, oxygen or sulfur, preferably nitrogen.

By the term "treating" and derivatives thereof as used herein, is meant prophylactic or therapeutic therapy.

By the term "organic molecule" and derivatives thereof as used herein, is meant the standard usage in the art to the ordinary organic chemist and as such excludes inorganic molecules and peptide molecules.

By the term "cell-membrane receptor" as used herein, is meant a protein macromolecule that spans the cell membrane and can transmit a signal from the extracellular media to the inside of the cell. Most natural cell-membrane receptors contain three regions: extracellular, transmembrane and intracellular domains. The extracellular domain serves as recognition site for specific ligands, the transmembrane domain acts as an anchor of the receptor to the cell membrane, and the intracellular domain typically contains recognition sequences that transduce the external signal to the inside of the cells. Further cell-membrane receptors within this invention can be comprised of only two domains: the transmembrane domain, typically a region of about 18-30 aminoacids with an overall nonpolar nature, and the intracellular domain, typically a region of 40-300 amino acids containing recognition sequences involved in signal initiation, such as the Box I, Box2 and tyrosine residues found in cytokine receptors (see Fukunaga et al., Cell, 1993, 74, 1079; Tanner et al, J. Biol. Chem. 1995, 270, 6523; Gurney et al., Proc Nat. Acad. Sci. USA 1995, 92, 5292).

All of the receptors within this invention have at least one point mutation.

By the term "metal-chelating receptor agonists", and derivatives thereof, as used herein means a small organic molecule having a molecular weight from about 100 to about 850, preferably having a molecular weight from about 200 to about 750, most preferably having a molecular weight from about 300 to about 650 and having from 1 to 4 metal binding motifs, preferably having one or two metal binding motifs. In one embodiment, metal chelation forms a symmetrical multimer, such as a dimer, of the receptor binding moiety.

By the term "metal binding motif", and derivatives thereof, as used herein means a continuation of atoms within a receptor binding moiety that have the following characteristics:
1) each continuation consist of 3 to 10 atoms, preferably 4 to 8 atoms, most preferably 4 or 5 atoms,
2) each continuation further consisting of two or more heteroatoms, preferably from 2 to 4 heteroatoms, most preferably 2 to 3 heteroatoms, preferably at least one of the heteroatoms is nitrogen, wherein the heteroatoms are separated from each other by one to four additional atoms selected from the group consisting of carbon, nitrogen, sulfur and oxygen, preferably carbon or nitrogen, preferably by 2 to 4 additional atoms, most preferably by 2 or 3 additional atoms, and
3) the configuration of heteroatoms within the metal binding motif allows for chelate coordination to a metal ion, such as a zinc (II), copper(II), nickel(II), iron(II), cobalt(II), manganese(II) ions, by providing for the formation of at least two coordinate bonds, preferably two or three coordinate bonds, simultaneously to a metal ion.

Examples of metal binding motifs for use in the present invention include but are not limited to the following: -N-C-C-N-, -N-C=C-N-, -N-C-C=N-, -N=C-C=N-, -O-C-C-N-, -O-C=C-N-, -O-C-C=N-, -O=C-C=N-, -S-C-C-N-, -S-C=C-N-, -S-C-C=N-, -S=C-C=N-, - S-C-C-S-, -N=C-N-N-, -N-C-N-N-, -O=C-N-N-, -S=C-N-N-, -O-C-C=O-, -O-N-C=O-, - O=C-C=N-N-, -N=C-N-C=N-, -O=C-N-C=N-, -N=C-C-C=N-, -O-C=C-C=O-, -N-C-C-C-N-, -N-C-C=C-N-, -N=C-C=C-N-, -N=C-C=C-O-, -N-C-C=C-O-, -N=C-C=C-S-, -S=C-C=C-S-, -O=C-N-C=N-, -N-N-C-C=N-, -N-N-C-N-N-, -N-C=N-C=N-, -N=C-N-C=N-C-C-N- and -N=C-N-C=N-C-C=N-. Further, the preferred metal binding motifs of the invention can be included as part of a combination. For example, the 8 atom zinc binding motif, - N=C-N-C=N-C-C=N-, in essentially an overlap of a 5 atom metal binding motif (that is - N=C-N-C=N-) and a 4 atom metal binding motif (that is -N-C-C=N-).

Preferred receptor binding moieties of the present invention comprise one or more of the following functional groups, preferably one or two of the following functional groups: 4-hydrazono-5-pyrazolones, 2-hydrazinophenols, 1-(2'-hydroxyphenyl)-thiosemicarbazones, 2-aryl-9-hydroxy-1H-naptho[1,2-d]imidazoles, 2-guanidinobenzimidazoles, 2-guanidinobenzoxazoles, 2-guanidionbenzothiazole, 2-mercaptomethylpyridines, acylacetones, acylhydrazines, 2-aminoethanethiols, 2-(imidazol-4-yl)ethylamines, 2-(imidazol-2-yl)ethylamines, 2-(imidazol-4-yl)ethylimines, 2-(imidazol-2-yl)ethylimines, 2-picolylamine, 8-hydroxyquinolines, 8-aminoquinolines, 8-mercaptoquinolines, ethylenediamines, pyridine-2-carboxaldimines, 2,2'-bipyridyls, 2-thiobenzaldimines, 2-hydroxybenzaldimines and 3'-{N'-[1-aryl-5-oxo-1,5-dihydropyrazol-4-ylidene]hydrazino)-2'-hydroxybiphenyl-3-carboxylic acids.

The above functional groups will generally form part of a larger molecule and may be further substituted in the formation of a receptor binding moiety. Preferred substituents for optional use on the above functional groups consist of one or more groups selected from the following: alkyl, aryl, hydroxy, alkoxy, acyloxy, carbamoyl, amino, N-acylamino, ketone, halogen, cyano, thio, carboxy and carboxamido.

International Application No. PCT/US99/30371, published as WO 00/35446 on June 22, 2000, discloses that the TPO receptor can be activated by small molecule ligands of Formula (I). To evaluate the role of the TPO receptor (Vigon et al. Proc. Natl. Acad. Sci. USA 1992, 89, 5640-5644) in compound action, we tested whether a cell line that did not respond to compounds could be rendered sensitive to compounds by expression of human TPO receptor. When transfected with a STAT-responsive luciferase reporter, cells from the human hepatoma cell line HepG2 (Aden et al. Nature, 1979, 282, 615) do not respond to TPO or compounds of Formula (I), but when cotransfected with a human TPO receptor expression vector and a reporter, the cells show a large increase in luciferase expression upon TPO treatment. Further, the cells also become responsive to the small-molecule agonists of Formula (I), with an activity pattern that was dependent on expression of the TPO receptor, indicating that the site of action of these compounds is the receptor itself.

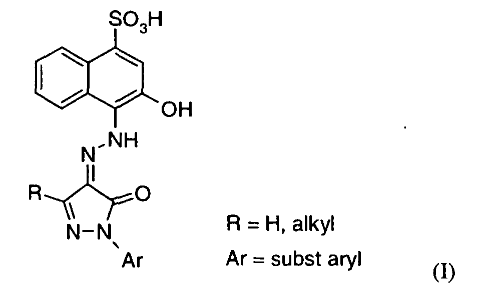

To further investigate the activity of compounds of Formula (I), cDNA clones for cynomolgous monkey TPO receptor were isolated and sequenced. Surprisingly, there was no activity detected by the compounds of Formula (I) against the cyno TPO receptor, despite the high degree of amino acid identity in its sequence when compared to that of the human receptor (96.5%). It was clear from these experiments that the compounds of Formula 1 show strict specificity for the human TPO receptor, a finding also confirmed by STAT activation assays of platelets from human and cyno origin. In order to examine the region of the TPO receptor required for compound activity, a series of human/cyno TPO-R chimeric receptors were transfected into HepG2 cells and tested for their response to compounds of Formula (I). Results from these studies showed that the compounds were active on chimeras that have the human transmembrane domain, and not on those derived from a cyno transmembrane domain. Comparison of the transmembrane sequences of human and cyno TPO receptors reveals a single amino acid difference, histidine at the amino acid position 499 in the human receptor is changed to leucine in the cyno. To investigate the role of this specific transmembrane domain mutation on the activity of compounds of Formula (I), two point mutations were constructed: a) a H499L change into the human TPO receptor transmembrane domain, creating a receptor with human extracellular and cytoplasmic domains and a cyno receptor transmembrane domain and b) a L499H change into the cyno receptor transmembrane domain creating a chimera with cyno extracellular and cytoplasmic domains and a human transmembrane domain. The H499L point mutation in the human TPO receptor rendered the receptor unresponsive to compounds of Formula (I), but had little effect on the response to TPO. Conversely, the L499H mutation in the cyno TPO receptor allowed it to be activated by both TPO and the compounds with potencies and efficacies similar to those seen on wild type human receptor. This single amino acid difference in the transmembrane domain was found to be responsible for the species specificity of this series. In analogy to the cyno receptor, the murine TPO receptor also has a leucine at the position equivalent of human Leu499 in the transmembrane domain and, as expected, it is also unresponsive to the action of compounds of Formula (I).

In another example of the invention, the murine G-CSFR was selected to test if the introduction of a His in the transmembrane region would render the receptor responsive to the action of compounds of Formula (I). As shown in Formula (II), the mGCSF-R (Fukunaga et al. Cell, 1990, 61, 341-350) does not contain a His in the transmembrane region and, consequently, was unresponsive to the effects of the compounds when transfected in into HepG2 cells.

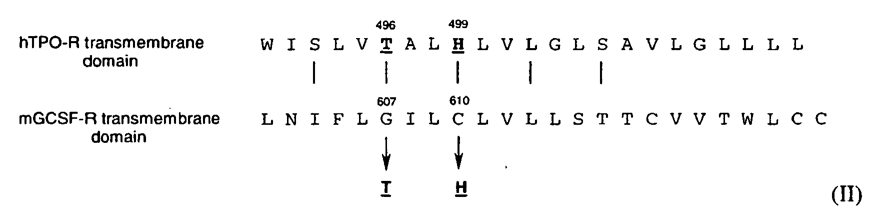

However, as shown in Fig 1 when the transmembrane Cys610 was changed to a His, the resulting mGCSF-R mutant became responsive to the action of compounds of Formula (I). The double mutant G607T / C610H mG-CSFR also became responsive to the action of the compounds, and showed higher levels of sensitivity than the corresponding C610H single mutant. These studies indicate that introduction of a histidine residue in the transmembrane domain of single-pass cell-surface receptors renders them responsive to their activation by compounds of Formula (I). Further, this effect can be amplified by the concomitant introduction of a threonine, preferably, three amino acids upstream from the histidine. This effect is mediated by metal ions, as demonstrated by the fact that no activation took place in the presence of a metal chelating agent such as EDTA (see Fig 1). The metal ion-dependence indicates the formation of a metal complex between compounds of Formula (I) and the receptor involving the His and Thr residues from the transmembrane domain. This complex, in turn, results in receptor activation by aggregation of receptor subunits on the surface of the cell.

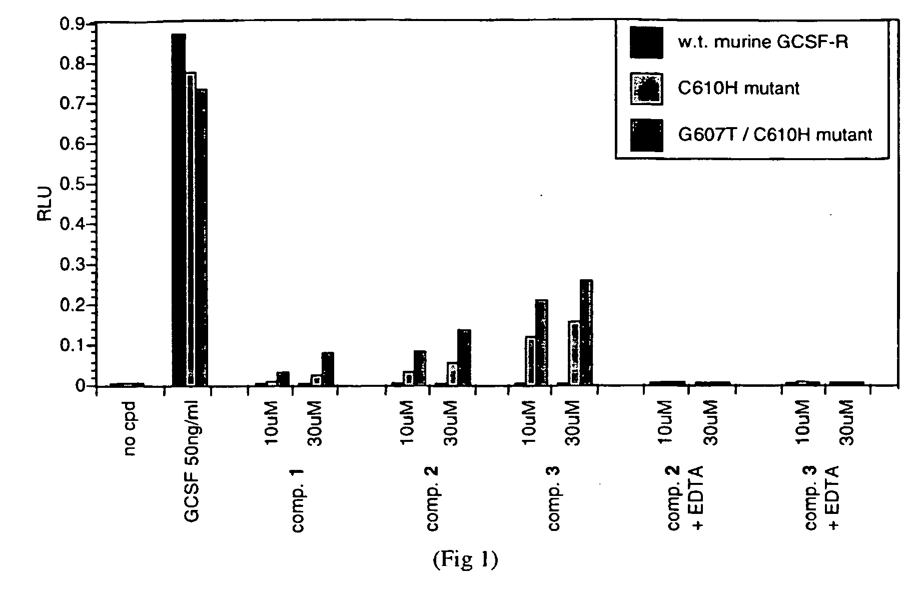

In determining the efficacy and potency of the presently invented compounds as agonists of dimeric cell-surface receptors, a luciferase reporter gene assay configured in HepG2 cells was utilized (see Tian et al., Science 1998, 281, 257-259).

Without further elaboration, it is believed that one skilled in the art can, using the proceeding description, utilize the present invention to its fullest extent. The following Examples are, therefore, to be construed as merely illustrative and not a limitation of the scope of the present invention in any way.

### Experimental Details

### Example 1

### TPO-R Plasmid Constructs:

Human Tpo receptor (hMPL) was cloned from HEL 92.1.7 cells (ATCC, Rockville, MD) by PCR (forward primer: 5'-ACG AAG CTT AGC CAA GAT GTC TCC TTG CTG GCA T-3' and reverse primer: 5'-AGC CTC GAG TCA AGG CTG CTG CCA ATA GCT-3'). The cloned full-length cDNA was confirmed by DNA sequencing analysis, then subcloned into HindIII-XhoI digested pSSF vector. The cynomolgous Tpo-R (cMPL) was cloned from bone marrow cDNA using the human 5' and 3' terminal coding sequences. The full-length cDNA was sequenced and subcloned into EcoRI digested pCDNA3.1 (+) vector (Invitrogen, San Diego, CA). Using the hMPL cDNA as a template, the BamHI-EcoRI fragment (nucletides 787-1836) was replaced with the corresponding cynomolgous fragment to generate h/cMPL-1 and the SacI-EcoRI fragment (nucleotides 1400-1836) was replaced with the corresponding cynomolgous fragment to generate h/cMPL-2. The c/hMPL construct was generated by replacing the BamHI-XhoI fragment (nucleotides 787-1836) of the cMPL cDNA with the corresponding human fragment. Both the T(TMt)G and T(TMg)G constructs were cloned by bridge PCR. For T(TMt)G construct, chimeric primers (forward primer: 5'-CTG GGC CTG CTG CTG CTG AGC CCC AAC AGG AAG AAT-3' and reverse primer: 5'-ATT CTT CCT GTT GGG GCT CAG CAG CAG CAG GCC CAG-3') were used to join the extracellular domain and transmembrane domain of hMPL sequence with the cytoplasmic domain of the hG-CSF-R sequence. Chimeric PCR primers (forward primer: 5'-GCC ACC GAG ACC GCC TGG ATC ATC CTG GGC CTG TTC-3' and reverse primer: 5'-GAA CAG GCC CAG GAT GAT CCA GGC GGT CTC GGT GGC-3') were used to generate T(TMg)G construct by joining the extracellular domain of the hMPL sequence with the transmembrane domain and cytoplasmic domain of the hG-CSF-R sequence. The transmembrane domain single point mutants, hMPL(TMc) and cMPL(TMh) were generated by incorporating the desired mutations into PCR primers and the cloned products were confirmed by sequence analysis.

### Example 2

### HepG2 Transfection:

HepG2 cells were plated in 24-well plates in triplicates for overnight. The cells were then transfected with the indicated receptor construct, a GAS response element containing luciferase reporter, 8x118-TkLUC and STAT5b expression vector (a gift of Dr. James Ihle, Memphis, TN) by Superfect method (QIAGEN, Valencia, CA) as instructed by the manufacturer. After an overnight recovery, the transfected cells were treated with 0.1 %DMSO, TPO or compound of Example 4 at the indicated concentrations for 4-5 hrs. The cells were then lysed and the level of luciferase expression was measured by a plate reader.

### Example 3

### GCSF-R Plasmid Constructs:

The transmembrane of the murine GCSF receptor was mutated with the following primers: 1) 5' CTA AAG CAT GTT GGC ACA AC 3'; 2) 5' CAT CTG ACC AGA AGG AAG TC 3'; 3) 5' AAC ATT TTC CTG ACC ATA CTT CAC TTA 3'; 4) 5' TAA GTG AAG TAT GGT CAG GAA AAT GTT 3'. Primers 1 and 2 were designed to replace a cystein residue at position 610 with a histidine. A second mutation was performed with primers 3 and 4 to replace the glycine residue at position 607 with a threonine. The mutagenesis was performed according to the manufacturer's recommendations using QuikChange, a site-directed mutagenesis kit from Stratagene (cat. number 200518-5). The transmenbrane region from eight clones from the His mutagenesis were sequenced using ThermoSequenase radiolabeled sequencing kit from USB (cat.79750). Seven clones were mutated. The receptor from one of the seven clones was sequenced in its entirety and proved to be correct. The gene was then transferred to a mammalian expression vector that had not gone through the mutagenesis protocol. The (His)GCSF-r was used to introduce the threonine mutation and produce the (His/Thr) double-mutant. Again 8 clones of the double mutant were screened for the mutation. All contained the His/Thr mutation. One of the double-mutants was fully sequenced and again transferred to an expression vector that had not gone through the mutagenesis procedure. The His and His/Thr GCSF-r plasmids were transfected overnight into HepG2 cells along with the 8x118Tk-Luc reporter using Lipofectamine Plus (GibcoBRL cat. 10964-013) following the manufacturer's protocol. The transfected cells were treated with compound or GCSF for 5 to 7hrs @ 37C in a humidified incubator. The cells were then lysed and assayed for luciferase production.

### Example 4

### Preparation of 3-Hydroxy-4-[(1-hydroxy-2-naphthalenyl)azo]-1-naphthalenesulfonic acid (compound 1)

The title compound is commercially available from Aldrich Chemical Company, Milwaukee, WI and used as provided. MS(ES) m/z 393 [M-H].

### Example 5

### Preparation of 3'-{N'-[1-(3,4-dimethylphenyl)-3-methyl-5-oxo-1,5-dihydropyrazol-4-ylidene]hydrazino)-2'-hydroxybiphenyl-3-carboxylic acid (compound 2)

### a) 2-Bromo-6-nitrophenol

2-Bromophenol (34.6 g, 0.2 mol) was added slowly to a cold (10°C) solution of sodium nitrate (30.5 g, 0.3 6 mol) in conc. sulfuric acid (42 g) and water (74 mL) and the resulting mixture was allowed to stir at room temperature for 2h. Water (210 mL) was added and the resulting mixture was extracted with diethyl ether and the extract was dried (MgSO₄), filtered and concentrated. The residue was purified by flash chromatography (silica gel, 10% ethyl acetate/hexanes) to afford first the title compound (10.9 g; 25%) as a bright, yellow solid. ¹H NMR (300 MHz, CDCl₃) δ 11.10 (S, I h), 8.13 (d, J = 7.9 Hz, 1H), 7.89 (d, J = 7.9 Hz, 1H), 6.90 (t, J = 7.9 Hz, 1H).

### b) 2-Bromo-6-nitroanisole

A mixture of the compound from Example 5a) (10.8 g; 0.0495 mol.), methyl iodide (3.4 mL; 0.00545 mol.) and potassium carbonate (8.2 g; 0.0592 mol.) in acetone (250 mL) was stirred and heated under reflux for 24h. The mixture was evaporated and the residue triturated with water to afford the title compound (8.7 g; 76%). mp 55-56°C. ¹H NMR (300 MHz, CDCl₃ δ 7.81-7.74 (m, 2H), 7.13 (t, J = 8.1 Hz, 1H), 4.02 (s, 3H); Anal. (C₇H₆NO₃Br) calcd: C, 36.24; H, 2.61; N, 6.04. found: C, 36.30; H, 2.59; N, 5.73.

### c) 2'-Methoxy-3'-nitrobiphenyl-3-carboxylic acid

A solution of the compound from Example 5b) (4.06 g, 17.5 mmol.), 3-carboxyphenylboronic acid (3.04 g, 18.4 mmol.), 2M aqu. sodium carbonate (17.5 mL; 35 mmol.) and tetrakistriphenylphosphino palladium(0) (875 mg) in 1,4-dioxane (105 mL) was stirred and heated under reflux under a nitrogen atmosphere for 24h.

The reaction mixture was cooled and evaporated and the residue treated with 6M aqu. hydrochloric acid (150 mL). The grey precipitate was filtered and washed well with water then diethyl ether to afford the title compound (2.13g; 47%) as a tan powder. ¹H NMR (300 MHz, d₆-DMSO) δ 8.12 (s, 1H), 8.03 (d, J = 7.9 Hz, 1H), 7.94 (dd, J = 7.9 Hz, 1.5 Hz, 1H), 7.85 (d, J = 7.9 Hz, 1H), 7.76 (dd, J = 7.5, 1.5 Hz, 1H), 7.66 (t, J = 7.5 Hz, 1H), 7.46 (t, j = 7.9 Hz, 1H), 3.46 (s, 3H).

### d) 2'-Hydroxy-3'-nitrobiphenyl-3-carboxylic acid

A solution of the compound from Example 5c) (2.13 g; 0.0077 mol.) in glacial acetic acid (25.0 mL) and 48% aqu/ hydrobromic acid (25.0 mL) was stirred and heated under reflux for 5h. The mixture was cooled and filtered to afford the title compound (1.57 g; 79%) as a tan powder. ¹H NMR (300 MHz, d₆-DMSO) δ 13.90 (s, 1H), 10.66 (s, 1H), 8.12 (t, J = 1.7 Hz, 1H), 8.07 (dd, J = 8.4, 1.7 Hz, 1H), 7.98 (dt, 7.8, 1.5 Hz, 1H), 7.79 (dt, J = 8.1, 1.7 Hz, 1H), 7.74 (dd, J = 7.5, 1.7 Hz, 1H), 7.62 (t, J = 7.8 Hz, 1H), 7.17 (dd, J = 8.4, 7.5 Hz, 1H).

### e) 4-Amino-3'-hydroxybiphenyl-3-carboxylic acid, hydrochloride salt

A solution of the compound from Example 5d) (1.71 g, 6.6 mmol.) in ethanol (75.0 mL), water (50.0 mL) and 3M aqu. sodium hydroxide (2. 1 mL, 6.8 mmol.) was hydrogenated over 10% palladium on carbon (210 mg) at room temperature and 50 psi for 2h. The reaction mixture was filtered, treated with 3M aqu. hydrochloric acid (25.0 mL) then evaporated and the residue triturated with a little water to afford the title compound (1.51 g; 100%) as a brown solid.11.3-8.7 (br s, 4H), 8.08 (s, 1H), 7.95 (d, J = 7.8 Hz, 1H), 7.74 (d, J = 7.8 Hz, 1H), 7.61 (t, J = 7.8 Hz, 1H), 7.34 (dd, J = 7.8, 1.4 Hz, 1H), 7.24 (dd, J = 7.8, 1.3 Hz, 1H), 7.04 (t, J = 7.8 Hz, 1H).

### f) 1-(3,4-Dimethylphenyl)-3-methyl-3-pyrazolin-5-one

A solution of 3,4-dimethylphenylhydrazine hydrochloride (17.7 g; 0.1 mol.), ethyl acetoacetate (13.0 g; 0.1 mol.) and sodium acetate (8.2 g; 0.1 mol.) in glacial acetic acid (250 mL) was stirred and heated under reflux for 24h. The mixture was cooled and evaporated and the residue dissolved in diethyl ether (1L) and carefully washed with sat. aqu. sodium hydrogen carbonate (5 x 200 mL). The ethereal layer was evaporated to afford the title compound (15.4 g; 76%). ¹H NMR (300 MHz, d₆-DMSO) δ 11.30 (br s, 1H), 7.49 (d,J = 1.4Hz, 1H), 7.43 (dd, J = 8.2 Hz, 1H), 7.14 (d, J = 8.2 Hz, 1H), 5.31 (s, 1H), 2.20 (s, 3H), 2.22 (s, 3H), 2.08 (s, 3H); MS(ES) m/z 203 [M+H].

### g) 3'-{N'-[1-(3,4-dimethylphenyl)-3-methyl-5-oxo-1,5-dihydropyrazol-4-ylidene]hydrazino}-2'-hydroxybiphenyl-3-carboxylic acid, hydrate

A suspension of the compound from Example 5e) (89.0 mg; 0.39 mmol.) in 1M aqu. hydrochloric acid (1.3 mL) was cooled to 5°C then treated dropwise with a solution of sodium nitrite (28.4 mg; 0.41 mmol.) in water (0.45 mL). The yellow mixture was stirred at 5°C for a further 10 min. then treated in one portion with the compound from Example 5f) (78.2 mg, 0.39 mmol.) followed by the portion-wise addition of sodium hydrogen carbonate (160 mg; 1.95 mmol.) and ethanol (1.8 mL) ensuring the final pH of the reaction mixture is approximately 7-8. The red solution was then stirred at room temperature for 24h. The mixture was filtered to give a red solid which was slurried in water (4.5L) and then acidified with concentrated hydrochloric acid. Filtration afforded the title compound (0.055 g; 32%) as an orange solid. mp 228°C (dec.). ¹H NMR (300 MHz, d₆-DMSO) δ 13.76 (s, 1H), 13.12 (s, 1H), 9.70 (s, 1H), 8.14 (s, 1H), 7.97 (dd, J = 7.7 Hz, 1H), 7.81 (dd, J = 7.7 Hz, 1H), 7.74-7.60 (m, 5H), 7.22-7.13 (m, 3H), 2.34 (s, 3H), 2.27 (s, 3H), 2.23 (s, 3H); Anal. (C₂₅H₂₂N₄O₄.1.0 H₂O) calcd: C, 65.21; H, 5.25; N, 12.17. found: C, 65.60; H, 4.96; N, 12.04.

### Example 6

### Preparation of 4-{[1-(3,4-dimethylphenyl)-5-hydroxy-3-methyl-1H-pyrazol-4-yl]azo}-3-hydroxy-1-naphthalenesulfonic acid (compound 3)

To a stirring solution of 1-diazo-2-naphthol-4-sulfonic acid (13.3 g, 53.1 mmol) and compound from Example 5f) (10.7 g, 53.1 mmol) in water (170 mL), sodium bicarbonate (13.38 g, 159.2 mmol) was added slowly. The resulting solution was heated at 60°C with stirring overnight. The solution was cooled to room temperature, and was adjusted to pH = 1 with 3 N hydrochloride solution. The purple precipitate was isolated by filtration and washed with water to provide the title compound as a red solid (23.3 g; 97%). MS(ES) m/z 451 [M-H].

## Claims

1. A DNA construct encoding a cell-membrane receptor, whose transmembrane domain has been modified with point mutations to Thr and His, and where the Thr and His are three residues apart.

2. A DNA construct as claimed in claim 1, wherein the cell-membrane receptor is a mutant of murine G-CSFR (murine granulocyte-colony stimulating factor receptor) whose transmembrane Cys610 has been changed to a His (C610H) and whose G607 has been changed to a Thr (G607T), as shown in formula (II):

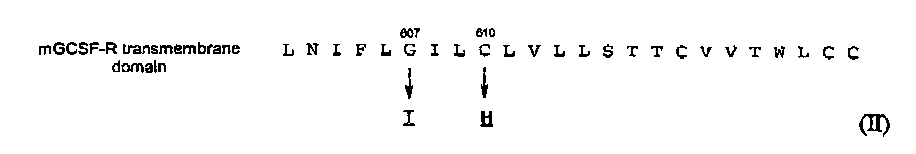

3. A cell-membrane receptor encoded by a DNA construct as defined in claim 1 or 2.

4. A genetically engineered cell containing and capable of expressing a DNA construct as defined in claim 1 or 2.

5. A cell as claimed in claim 4 containing a target gene under the expression control of a transcriptional control element responsive to the receptor of claim 3.

6. A method for activating a cell-membrane receptor which comprises exposing the cells as defined in claim 4 with a metal-chelating receptor agonist, wherein the cells are grown in a culture medium and the exposing is effected by adding the metal-chelating receptor agonist to the culture medium,
and wherein the metal-chelating receptor agonist is a small organic molecule having a molecular weight from 100 to 850, and having from 1 to 4 metal binding motifs, and wherein the metal binding motif(s) is or are selected from the following: - N-C-C-N-, -N-C=C-N-, -N-C-C=N-, -N=C-C=N-, -O-C-C-N-, -O-C=C-N-, -O-C-C=N-, -O=C-C=N-, -S-C-C-N-, -S-C=C-N-, -S-C-C=N-, -S=C-C=N-, -S-C-C-S-, -N=C-N-N-. - N-C-N-N-, -O=C-N-N-, -S=C-N-N-, -O-C-C=O-, -O-N-C=O-, -O=C-C=N-N-, -N=C-N-C=N-, -O=C-N-C=N-, -N=C-C-C=N-, -O-C=C-C=O-, -N-C-C-C-N-, -N-C-C=C-N-, - N=C-C=C-N-, -N=C-C=C-O-, -N-C-C=C-O-, -N=C-C=C-S-, -S=C-C=C-S-, -O=C-N-C=N-, -N-N-C-C=N-, -N-N-C-N-N-, -N-C=N-C=N-, -N=C-N-C=N-C-C-N- and -N=C-N-C=N-C-C=N-.

7. The use of a metal-chelating receptor agonist in the preparation of a pharmaceutical composition which contains the metal-cheating receptor agonist for administration in a therapeutically effective dose in a pharmaceutically acceptable carrier,
wherein the composition is for activating a cell-membrane receptor by a method which comprises exposing the cells as defined in claim 4 with the metal-chelating receptor agonist,
wherein the cells are present in a host organism which is a mammal, and wherein the exposing is effected by administering the metal-chelating receptor agonist to the host organism,
and wherein the metal-chelating receptor agonist is a small organic molecule having a molecular weight from 100 to 850, and having from 1 to 4 metal binding motifs,
and wherein the metal binding motif(s) is or are selected from the following: -N-C-C-N-, -N-C=C-N-, -N-C-C=N-, -N=C-C=N-, -O-C-C-N-, -O-C=C-N-, -O-C-C=N-, -O=C-C=N-, -S-C-C-N-, -S-C=C-N-, -S-C-C=N-, -S=C-C=N-, -S-C-C-S-, -N=C-N-N-, -N-C-N-N-, -O=C-N-N-, -S=C-N-N-, -O-C-C=O-, -O-N-C=O-, -O=C-C=N-N-, -N-C-N-C=N-, -O=C-N-C=N-, -N=C-C-C=N-, -O-C=C-C=O-, N-C-C-C N-, -N-C-C=C-N-, -N=C-C=C-N-, -N=C-C=C-O-, -N-C-C=C-O-, -N=C-C==C-S-, -S=C-C=C-S-, -O=C-N-C=N-, N-N-C-C=N-, -N-N-C-N-N-, -N-C=N-C=N-, -N=N-C=N-C-C-N- and -N=C-N-C=N-C-C=N-.

8. Use as claimed in claim 7, wherein the metal-chelating receptor agonist has one or two metal binding motifs.

9. Use as claimed in claim 7 or 8, wherein receptor binding moieties comprise one or two of the following functional groups (optionally substituted): 4-hydrazono-5-pyrazolones, 2-hydrazinophenols, 1-(2'-hydroxyphenyl)-thiosemicarbazones, 2-aryl-9-hydroxy-1H-naptho[1,2-d]imidazoles, 2-guanidinobenzinudazoles, 2-guanidinobenzoxazoles, 2-guanidionbenzothiazole, 2-mercaptomethylpyridines, acylacetones, acylhydrazines, 2-aminoethanethiols, 2-(imidazol-4-yl)ethylamines, 2-(imidazol-2-yl)ethylamines, 2-(imidazol-4-yl)ethylimines, 2-(imidazol-2-yl)ethylimines, 2-picolylamine, 8-hydroxyquinolines, 8-aminoquinolines, 8-mercaptoquinolines, ethylenediamines, pyridine-2-carboxaldimines, 2,2'-bipyridyls, 2-thiobenzaldimines, 2-hydroxybenzaldimines, and 3'-{N'-[1-aryl-5-oxo-1,5-dihydropyrazol-4-ylidene]hydrazino)-2'-hydroxybiphenyl-3-carboxylic acids.

10. Use as claimed in claim 7, 8 or 9, wherein the metal-chelating receptor agonist is a compound of Formula (I):

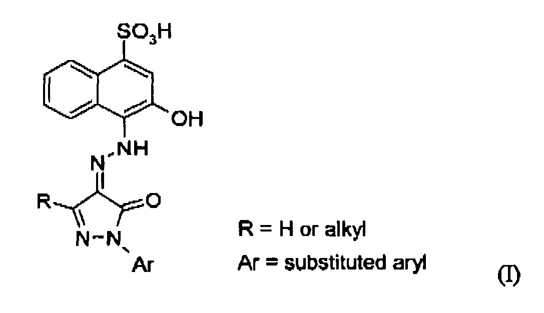

11. The use as claimed in claim 7, 8, 9 or 10 wherein the composition containing the metal-chelating receptor agonist is for oral administration.

12. The use as claimed in claim 7, 8, 9 or 10 wherein the composition containing the metal-chelating receptor agonist is for parenteral administration.

13. A kit which comprises at least one DNA construct as defined in claim 1 or 2.

14. A kit as claimed in claim 13 which further comprises a metal-chelating receptor agonist as defined in claim 7, 8, 9 or 10.

## Patentansprüche

1. DNA-Konstrukt, das einen Zellmembranrezeptor codiert, dessen Transmembrandomäne durch Punktmutationen zu Thr und His modifiziert worden ist, und wobei das Thr und His drei Reste auseinanderliegen.

2. DNA-Konstrukt gemäß Anspruch 1, worin der Zellmembranrezeptor eine Mutante des murinen G-CSFR (muriner Granulozyten-Kolonien stimulierender Faktor-Rezeptor) ist, dessen transmembranes Cys610 zu His (C610H) geändert worden ist, und dessen G607 zu einem Thr (G607T) geändert worden ist, wie in Formel (II) gezeigt:

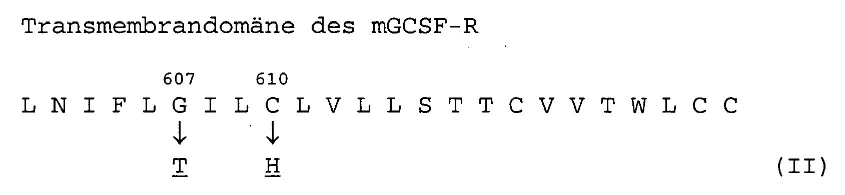

3. Zellmembranrezeptor, der durch ein DNA-Konstrukt gemäß Anspruch 1 oder 2 codiert wird.

4. Genetisch manipulierte Zelle, die ein DNA-Konstrukt gemäß Anspruch 1 oder 2 enthält und in der Lage ist, dieses zu exprimieren.

5. Zelle gemäß Anspruch 4, die ein Zielgen enthält, das unter der Expressionskontrolle eines transkriptionellen Kontrollelements steht, das auf den Rezeptor des Anspruchs 3 reagiert.

6. Verfahren zum Aktivieren eines Zellmembranrezeptors, das das Exponieren der Zellen gemäß Anspruch 4 mit einem metallchelatbildenden Rezeptoragonisten umfaßt, worin die Zellen in einem Kulturmedium kultiviert werden und das Exponieren durch Zugabe des metallchelatbildenden Rezeptoragonisten zu dem Kulturmedium erfolgt,
und worin der metallchelatbildende Rezeptoragonist ein kleines organisches Molekül mit einem Molekulargewicht von 100 bis 850 und mit 1 bis 4 metallbindenden Motiven ist, und worin das/die metallbindende(n) Motiv(e) aus den folgenden ausgewählt ist/sind:
- N-C-C-N-, -N-C=C-N-, -N-C-C=N-, -N=C-C=N-, -O-C-C-N-, -O-C=C-N-, -O-C-C=N-, -O=C-C=N-, -S-C-C-N-, -S-C=C-N-, -S-C-C=N-, -S=C-C=N-, -S-C-C-S-, -N=C-N-N-, -N-C-N-N-, -O=C-N-N-, -S=C-N-N-, -O-C-C=O-, -O-N-C=O-, -O=C-C=N-N-, -N=C-N-C=N-, -O=C-N-C=N-, -N=C-C-C=N-, -O-C=C-C=O-, -N-C-C-C-N-, -N-C-C=C-N-, -N=C-C=C-N-, -N=C-C=C-O-, -N-C-C=C-O-, -N=C-C=C-S-, -S=C-C=C-S-, -O=C-N-C=N-, -N-N-C-C=N-, -N-N-C-N-N-, -N-C=N-C=N-, -N=C-N-C=N-C-C-N-und -N=C-N-C=N-C-C=N-.

7. Verwendung eines metallchelatbildenden Rezeptoragonisten bei der Herstellung einer pharmazeutischen Zusammensetzung, die den metallchelatbildenden Rezeptoragonisten für die Verabreichung in einer therapeutisch effektiven Dosis in einem pharmazeutisch annehmbaren Träger enthält,
worin die Zusammensetzung der Aktivierung eines Zellmembranrezeptors durch ein Verfahren dient, das das Exponieren der Zellen gemäß Anspruch 4 mit dem metallchelatbildenden Rezeptoragonisten umfaßt,
worin die Zellen in einem Wirtsorganismus vorliegen, der ein Säugetier ist, und worin das Exponieren durch Verabreichen des metallchelatbildenden Rezeptoragonisten an den Wirtsorganismus erfolgt,
und worin der metallchelatbildende Rezeptoragonist ein kleines organisches Molekül mit einem Molekulargewicht von 100 bis 850 und mit 1 bis 4 metallbindenden Motiven ist, und worin das/die metallbindende(n) Motiv(e) aus den folgenden ausgewählt ist/sind:
- N-C-C-N-, -N-C=C-N-, -N-C-C=N-, -N=C-C=N-, -O-C-C-N-, -O-C=C-N-, -O-C-C=N-, -O=C-C=N-, -S-C-C-N-, -S-C=C-N-, -S-C-C=N-, -S=C-C=N-, -S-C-C-S-, -N=C-N-N-, -N-C-N-N-, -O=C-N-N-, -S=C-N-N-, -O-C-C=O-, -O-N-C=O-, -O=C-C=N-N-, -N=C-N-C=N-, -O=C-N-C=N-, -N=C-C-C=N-, -O-C=C-C=O-, -N-C-C-C-N-, -N-C-C=C-N-, -N=C-C=C-N-, -N=C-C=C-O-, -N-C-C=C-O-, -N=C-C=C-S-, -S=C-C=C-S-, -O=C-N-C=N-, -N-N-C-C=N-, -N-N-C-N-N-, -N-C=N-C=N-, -N=C-N-C=N-C-C-N-und -N=C-N-C=N-C-C=N-.

8. Verwendung gemäß Anspruch 7, worin der metallchelatbildende Rezeptoragonist ein oder zwei metallbindende Motive hat.

9. Verwendung gemäß Anspruch 7 oder 8, worin die Rezeptorbindungsreste eine oder zwei der folgenden funktionellen Gruppen (gegebenenfalls substituiert) umfassen: 4-Hydrazono-5-pyrazolone, 2-Hydrazinophenole, 1-(2'-Hydroxyphenyl)thiosemicarbazone, 2-Aryl-9-hydroxy-1H-naphtho[1,2-d]imidazole, 2-Guanidinobenzimidazole, 2-Guanidinobenzoxazole, 2-Guanidinobenzothiazole, 2-Mercaptomethylpyridine, Acylacetone, Acylhydrazine, 2-Aminoethanthiole, 2-(Imidazol-4-yl)ethylamine, 2-(Imidazol-2-yl)ethylamine, 2-(Imidazol-4-yl)ethylimine, 2-(Imidazol-2-yl)ethylimine, 2-Picolylamin, 8-Hydroxychinoline, 8-Aminochinoline, 8-Mercaptochinoline, Ethylendiamine, Pyridin-2-carboxaldimine, 2,2'-Bipyridyle, 2-Thiobenzaldimine, 2-Hydroxybenzaldimine und 3'-{N'-[1-Aryl-5-oxo-1,5-dihydroypyrazol-4-yliden]hydrazino}-2'-hydroxybiphenyl-3-carbonsäuren.

10. Verwendung gemäß Anspruch 7, 8 oder 9, worin der metallchelatbildende Rezeptoragonist eine Verbindung der Formel (I) ist:

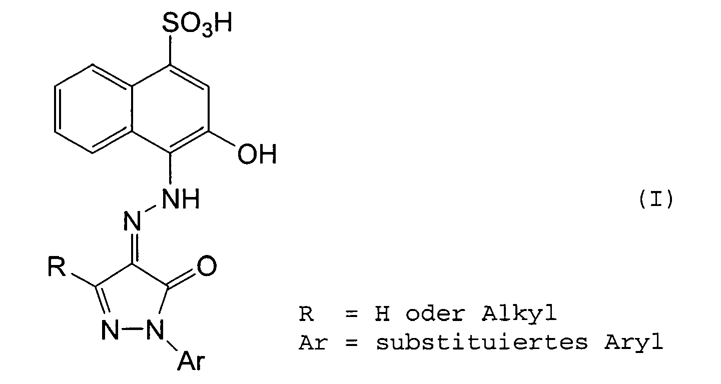

11. Verwendung gemäß Anspruch 7, 8, 9 oder 10, worin die Zusammensetzung, die den metallchelatbildenden Rezeptoragonisten enthält, für die orale Verabreichung ist.

12. Verwendung gemäß Anspruch 7, 8, 9 oder 10, worin die Zusammensetzung, die den metallchelatbildenden Rezeptoragonisten enthält, für die parenterale Verabreichung ist.

13. Kit, das wenigstens ein DNA-Konstrukt gemäß Anspruch 1 oder 2 umfaßt.

14. Kit gemäß Anspruch 13, das ferner einen metallchelatbildenden Rezeptoragonisten gemäß Anspruch 7, 8, 9 oder 10 umfaßt.

## Revendications

1. Construction d'ADN codant un récepteur de membrane cellulaire, dont le domaine transmembranaire a été modifié par des mutations ponctuelles en Thr et His, et où les Thr et His sont espacés de trois résidus.

2. Construction d'ADN selon la revendication 1, dans laquelle le récepteur de membrane cellulaire est un mutant de G-CSFR murin (récepteur du facteur stimulant le développement des granulocytes murin) dont le Cys610 transmembranaire a été changé en un His(C610H) et dont le G607 a été changé en un Thr(G607T), de la façon qui est représentée dans la formule (II) :

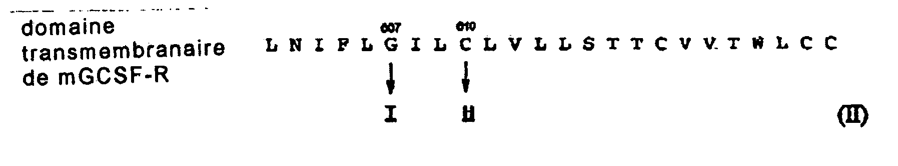

3. Récepteur de membrane cellulaire codé par une construction d'ADN selon la revendication 1 ou 2.

4. Cellule modifiée par génie génétique contenant une construction d'ADN selon la revendication 1 ou 2 et capable d'exprimer celle-ci.

5. Cellule selon la revendication 4 contenant un gène cible sous le contrôle d'expression d'un élément de contrôle transcriptionnel sensible au récepteur de la revendication 3.

6. Procédé pour activer un récepteur de membrane cellulaire qui comprend l'exposition des cellules selon la revendication 4 à un agoniste de récepteur chélateur de métal, dans lequel les cellules sont cultivées dans un milieu de culture et l'exposition est effectuée par addition de l'agoniste de récepteur chélateur de métal au milieu de culture,
et dans lequel l'agoniste de récepteur chélateur de métal est une petite molécule organique ayant un poids moléculaire de 100 à 850, et ayant de 1 à 4 motifs de liaison de métal, et dans lequel le ou les motifs de liaison de métal sont choisis parmi les suivants :
- N-C-C-N-, -N-C=C-N-, -N-C-C=N-, -N=C-C=N-, -O-C-C-N-, -O-C=C-N-, -O-C-C=N-, -O=C-C=N-, -S-C-C-N-, -S-C=C-N-, -S-C-C=N-, -S=C-C=N-, -S-C-C-S-, -N=C-N-N-, -N-C-N-N-, -O=C-N-N-, -S=C-N-N-, -O-C-C=O-, -O-N-C=O-, -O=C-C=N-N-, -N=C-N-C=N-, -O=C-N-C=N-, -N=C-C-C=N-, -O-C=C-C=O-, -N-C-C-C-N-, -N-C-C=C-N-, -N=C-C=C-N-, -N=C-C=C-O-, -N-C-C=C-O-, -N=C-C=C-S-, -S=C-C=C-S-, -O=C-N-C=N-, -N-N-C-C=N-, -N-N-C-N-N-, -N-C=N-C=N-, -N=C-N-C=N-C-C-N- et -N=C-N-C=N-C-C=N-.

7. Utilisation d'un agoniste de récepteur chélateur de métal dans la préparation d'une composition pharmaceutique qui contient l'agoniste de récepteur chélateur de métal destinée à l'administration d'une dose efficace du point de vue thérapeutique dans un support acceptable du point de vue pharmaceutique,
dans laquelle la composition est destinée à activer un récepteur de membrane cellulaire par un procédé qui comprend l'exposition des cellules selon la revendication 4 à l'agoniste de récepteur chélateur de métal,
dans laquelle les cellules sont présentes dans un organisme hôte qui est un mammifère et dans laquelle l'exposition est effectuée par administration de l'agoniste de récepteur chélateur de métal à l'organisme hôte,
et dans laquelle l'agoniste de récepteur chélateur de métal est une petite molécule organique ayant un poids moléculaire de 100 à 850, et ayant de 1 à 4 motifs de liaison de métal,
et dans laquelle le ou les motifs de liaison de métal sont choisis parmi les suivants :
- N-C-C-N-, -N-C=C-N-, -N-C-C=N-, -N=C-C=N-, -O-C-C-N-, -O-C=C-N-, -O-C-C=N-, -O=C-C=N-, -S-C-C-N-, -S-C=C-N-, -S-C-C=N-, -S=C-C=N-, -S-C-C-S-, -N=C-N-N-, -N-C-N-N-, -O=C-N-N-, -S=C-N-N-, -O-C-C=O-, -O-N-C=O-, -O=C-C=N-N-, -N=C-N-C=N-, -O=C-N-C=N-, -N=C-C-C=N-, -O-C=C-C=O-, -N-C-C-C-N-, -N-C-C=C-N-, -N=C-C=C-N-, -N=C-C=C-O-, -N-C-C=C-O-, -N=C-C=C-S-, -S=C-C=C-S-, -O=C-N-C=N-, -N-N-C-C=N-, -N-N-C-N-N-, -N-C=N-C=N-, -N=C-N-C=N-C-C-N- et -N=C-N-C=N-C-C=N-.

8. Utilisation selon la revendication 7, dans laquelle l'agoniste de récepteur chélateur de métal a un ou deux motifs de liaison de métal.

9. Utilisation selon la revendication 7 ou 8, dans laquelle les parties de liaison de récepteur comprennent un ou deux des groupes fonctionnels suivants (éventuellement substitués) : 4-hydrazono-5-pyrazolones, 2-hydrazinophénols, 1-(2'-hydroxyphényl)-thiosemicarbazones, 2-aryl-9-hydroxy-1H-naphto [1,2-d] imidazoles, 2-guanidinobenzimidazoles, 2-guanidinobenzoxazoles, 2-guanidinobenzothiazole, 2-mercaptométhylpyridines, acylacétones, acylhydrazines, 2-aminoéthanethiols, 2-(imidazol-4-yl)éthylamines, 2-(imidazol-2-yl)éthylamines, 2-(imidazol-4-yl)éthylimines, 2-(imidazol-2-yl)éthylimines, 2-picolylamine, 8-hydroxyquinoléines, 8-aminoquinoléines, 8-mercaptoquinoléines, éthylènediamines, pyridine-2-carboxaldimines, 2,2'-bipyridyles, 2-thiobenzaldimines, 2-hydroxybenzaldimines et acides 3'-{N'-[1-aryl-5-oxo-1,5-dihydropyrazol-4-ylidène]hydrazino}-2'-hydroxybiphényl-3-carboxyliques.

10. Utilisation selon la revendication 7, 8 ou 9, dans laquelle l'agoniste de récepteur chélateur de métal est un composé de Formule (I) :

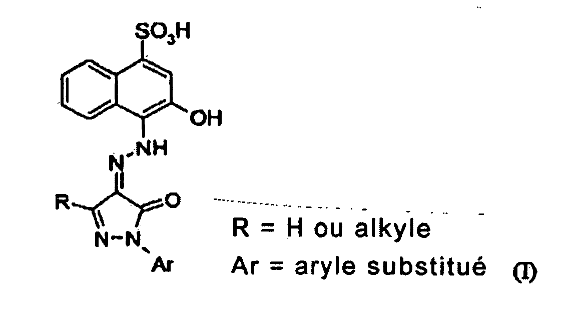

11. Utilisation selon la revendication 7, 8, 9 ou 10, dans laquelle la composition contenant l'agoniste de récepteur chélateur de métal est destinée à une administration orale.

12. Utilisation selon la revendication 7, 8, 9 ou 10, dans laquelle la composition contenant l'agoniste de récepteur chélateur de métal est destinée à une administration parentérale.

13. Trousse qui comprend au moins une construction d'ADN selon la revendication 1 ou 2.

14. Trousse selon la revendication 13, qui comprend de plus un agoniste de récepteur chélateur de métal selon la revendication 7, 8, 9 ou 10.
